# EUROPEAN PATENT APPLICATION

(11) **EP 1 915 987 A1**
(43) Date of publication of application: **30.04.2008**
(21) Application number: 06022538.0
(22) Date of filing: 27.10.2006
(51) Int. Cl.: A61K 9/16, A61K 9/19, A61K 9/127

(54) **Spray-freeze-drying process for the preparation of pellets comprising percolation drying**

(71) Applicant: MediGene AG, 82152 Planegg (DE); Ludwig-Maximilians-Universität München, 80539 München (DE)
(72) Inventor: Haas, Heinrich, 80337 München (DE); Drexler, Klaus, 82140 Olching (DE); Wiggenhorn, Michael, 80801 München (DE); Winter, Gerhard, 82377 Penzberg (DE)
(74) Representative: Weiss, Wolfgang

(57) **Abstract**

The present invention relates to dry particles and methods of preparing the same, in particular to methods of producing dry particles comprising a thermally labile component such as a colloidal system or a thermally labile biopharmaceutical compound, to the use of such dry particles, and to pharmaceutical compositions comprising the same.

## Description

The present invention relates to dry particles and methods of preparing the same, in particular to methods of producing dry particles comprising a thermally labile component such as a colloidal system or a thermally labile biopharmaceutical agent, to the use of such dry particles, and to pharmaceutical compositions comprising the same.

In the pharmaceutical field, particles are prepared for a variety of applications such as pulmonary and nasal administration, as well as for reconstitution and injection. There are several classes of devices currently available for pulmonary drug administration using aerosols. These devices include nebulizers (drug in dissolved state aerosolised with compressed air), metered-dose inhalers (MDls) and dry powder inhalers (DPIs). This technology includes the preparation of drug carrier systems, which can be used as a dry powder or be reconstituted afterwards like ready-to-use formulations.

Systems like liposomes or solid lipid particles are examples of colloidal systems. Liposomes are artificial membranes composed of single or multiple bilayers enclosing an aqueous compartment. They form spontaneously when lipids are dispersed in an aqueous environment. Liposomes are spherical self-closed structures, composed of curved lipid bilayers, which enclose part of the surrounding solvent into their interior. Hydrophobic drugs and compounds can be incorporated into the lipid bilayers, hydrophilic drugs and compounds can be incorporated within their aqueous cores. Most liposomes are non-toxic, non-antigenic and biodegradable in character since they have the molecular characteristics of mammalian membranes.

However, aqueous liposome dispersions have limited physical and chemical stability. To a certain extent the physical stability can be improved by incorporation of charged lipids into the liposomes (cationic or anionic liposomes). However, this does not solve the problem of chemical stability. Therefore, the development of a dry particle formulation is frequently used.

Liposome formulations are mostly administrated by injection. Most present liquid liposome products are not stable over a longer storage period. Therefore liposome formulations are subjected to extremely stringent quality criteria, because they can undergo a variety of chemical and physical degradation processes. For pharmaceutical products it is desirable to have final formulations with a stability of at least six months to two years at room temperature or at refrigeration temperature. These factors restrict the use of liposomes as practical carriers of biologically active compounds. For liposomes, techniques for re- and dehydration have been developed to meet these requirements.

In fact, long term stability of liposome formulations is greatly enhanced when they are stored as dry rather than liquid formulations. A commonly used stabilization method for aqueous liposome suspensions by freeze-drying is described in US Pat. Nos. 4,229,360 and 4,247,411. Freeze-drying of the liposome components from a suitable organic solvent is described in US Pat. No. 4,311,712. These freeze-dried preparations result in a porous matrix which is easily hydrated. Although many formulations have been stabilized with the latter technology, it has some serious drawbacks. It is very time- and energy-consuming and therefore expensive especially in a batch process. The classical freeze-drying procedure results in a cake-like primary product, which has to be ground in an additional manufacturing step to obtain powder particles suitable for further handling.

The spray-drying method for preparing a stable liposome precursor in the form of a mixture of spray-dried liposomal components including one or more biologically active compounds which may be stored dry and reconstituted with water to form a liposomal preparation immediately prior to use is described in US Pat. No. 4,830,858. A major problem of the spray-drying technique for the dehydration of colloidal drug carrier systems like liposomes is the use of high temperatures. This is especially true for dispersions with high water content. Heat stress and oxidative stress can lead to degradation and decomposition of the drug and the lipid components.

Another solution suggested for overcoming the limited physical stability of liposomes is to prepare and store a film of the lipid/biologically active compound and to disperse the film just prior to administration to form the aqueous liposomal preparation. However, unit dose film preparation presents serious practical difficulties like the requirement of a container with a high surface area to facilitate solvent evaporation and deposition of a thin film suitable for rapid rehydration to form liposomes readily. This type of container by virtue of its bulk would present severe storage problems.

In another approach to overcome the drawbacks of the conventional spray-freeze-drying method, the whole process is separated into the individual steps of spray freezing and lyophilization. The dispersion of preformed frozen droplets is dried by lyophilization which is described in WO 03/087339. However, the problems of scalability and again the time consuming freeze-drying process are major drawbacks.

To decrease the drying times of frozen material, processes involving the fluidization of the material in a gas, forming a whirling layer or fluidized bed, have been developed. Drying in fluidized beds may be performed under ambient atmospheric pressure (US 3,313,032, US 3,436,837, US 4,608,764) or under vacuum conditions (US 3,269,025, US 6,584,782). The strong motion of the particles in said fluidized beds leads to undesired abrasion of the particles. Hence, material for the further processing of the drying material is lost. The fine, dust-like particulate material generated by abrasion has to be collected by elaborate filter systems. Particularly in manufacturing processes involving toxic substances, the formation of this fine particulate material should be avoided.

Thus, the problem underlying the present invention was to provide a method to prepare dry particles comprising a temperature-sensitive component.

The solution of the above problem is achieved by providing the embodiments characterized in the claims and further depicted in the description of the present invention.

A first aspect of the invention relates to a method for the preparation of dry particles from an aqueous medium comprising a colloidal system and/or a biopharmaceutical agent comprising the steps
a) providing said aqueous medium comprising a colloidal system and/or a biopharmaceutical agent,
b) freezing the aqueous medium of step a) to obtain frozen particles, and
c) dehydrating the frozen particles and/or pellets of step b) to obtain dry particles,
characterized in that step c) is performed by contacting the frozen particles under reduced pressure with a percolation medium under non-fluidizing conditions.

In a preferred embodiment, the percolation medium is passed over and/or through said frozen particles under non-fluidizing conditions. More preferably, step c) is performed without thawing.

The inventive method is particularly suitable for drying aqueous dispersions of colloidal drug carrier systems, such as liposomes or lipid complexes, or suspensions of biopharmaceutical agents, which are sensitive to chemical and/or physical degradation or modification. Preferred is the drying of dispersions of liposomes comprising an active pharmaceutical ingredient, in order to obtain dry particles which are stable and can easily be reconstituted to aqueous compositions.

The method according to the present invention allows the removal of the solvent under mild conditions in a reduced time and economic process, so that the structure of colloids and the activity of an active compound can be well preserved. Hence, the inventive method is advantageous for drying thermally labile compounds compared to spray-drying which requires higher temperatures.

In contrast to conventional lyophilization techniques which result in a porous cake-like product, the inventive method produces free-flowing particles which have favourable handling properties for the subsequent production steps like filling of vials.

Percolation of a cryogenic fluid such as a gas through the drying pellets under non-fluidizing conditions as performed in the inventive method is advantageous over the use of a fluidized bed (as described in US 3,269,025, US 3,313,032, US 3,436,837, US 4,608,764) because the strong movement of the particles in the fluidized bed leads to an undesired abrasion of material. Hence, losing material for the further processing is prevented. The avoidance of fine dust-like particles generated by abrasion is particularly advantageous in manufacturing processes involving toxic compounds. Furthermore, percolation decreases the drying time compared to conventional lyophilization.

In contrast to other methods which use a gas stream for the transport of moisture in the drying material at ambient pressure like US 3,313,032, US 3,436,837, US 4,608,764, the inventive method combines the use of low pressure and a medium flow for the transport of moisture, thereby decreasing process time and/or process temperature.

It is another object of the present invention to provide a product obtainable by the afore-described method. Typical products obtainable by the inventive method are dry, solid particles comprising a hydrophilic excipient which may be a water-soluble saccharide such as mannitol or trehalose. Preferably, the hydrophilic excipient is present in an amount of at least 50% by weight, more preferably at least 80% by weight, and most preferably at least 90% by weight based on the weight of the total product. At least part of these particles individually comprise one or more incorporated, optionally thermally labile, colloidal systems, such as liposomes. An active ingredient may be incorporated within, or associated with, the colloidal system. The colloidal system can be thermally labile either with regard to the chemical nature of one or more of the constituents, for example the active ingredient, of the colloid or to the physical properties of the system. The size of the dry particles and/or pellets is usually selected to be substantially larger than that of the colloidal system. For example, the dry particles and/or pellets may have an average diameter in the range of about 5 µm to about 5000 µm, whereas the incorporated colloidal system may be in the range of about 20 nm to about 5 µm.

The inventive process may be used for the manufacture of a medicament. The inventive dry particles may be used as such, or mixed with further ingredients, to form a pharmaceutical composition. Among the preferred types of pharmaceutical compositions are preparations for injection or infusion, which are obtained by reconstituting the particles with an appropriate liquid carrier, such as water for injection, sterile buffer or saline solution.

Another aspect of the invention relates to an apparatus to perform the inventive method.

### DEFINITIONS

"About" in the context of amount values refers to an average deviation of maximum +/-20%, preferably +/-10% based on the indicated value. For example, an amount of about 30 mol% cationic lipid refers to 30 mol% +/-6 mol% and preferably 30 mol% +/-3 mol% cationic lipid with respect to the total lipid/amphiphile molarity.
"Active compound" refers to a compound, or mixture of compounds, which has a particular bioactivity based on which it is useful as an agent useful for the diagnosis, prevention, or treatment of a human or animal disease or condition. Drug substances, diagnostic compounds and vaccines are important examples of active compounds according to the present invention.
"Aqueous medium" or "aqueous liquid" is a liquid material which contains water. The material may represent a single liquid phase, or a two- or multiphase system, wherein the continuous phase is liquid and contains water. An aqueous suspension or an dispersion consists of an aqueous continuous phase. An aqueous medium which contains a colloidal material is hereinafter sometimes referred to as an aqueous colloidal dispersion or solution.
"Biopharmaceutical agent" refers to a biological molecule, especially to a molecule derived from amino acids or nucleotides, such as a peptide, polypeptide, or a nucleic acid, e.g. a DNA, an RNA or a nucleic acid analog, and which is physiologically active when applied to a mammal, especially to a human patient, particularly in a pharmaceutical acceptable form.
"Camptothecins" refers to the chinoline-based alkaloid 4-Ethyl-4-hydroxy-1H-pyrano [3',4' : 6,7] indolizino [1,2-b] quinoline-3, 14 (4H, 12H)-dione (CAS 7689-03-4) that can be derived from Camptothecea acuminata, as well as derivatives and precursor molecules, like methoxylated analogs and carboxylated analogs, Topotecan, Irinotecan and SN38. Camptothecins are used for the treatment of cancer. Camptothecins are inhibitors of topoisomerase I.
"Colloidal system", "colloidal carrier system" or "colloidal drug carrier" is herein defined as a system of any composition which is a colloid and has a colloidal size range such as an average diameter of about 10 nm to about 10 µm. More preferably, the colloidal system has an average diameter of about 20 nm to about 5 µm, and particularly from about 50 nm to about 1 µm.
"Cryogenic fluid" is a material that is liquid in the temperature range that is necessary to freeze aqueous solutions, preferably at a temperature below -90°C.
"Derivative" refers to a compound derived from some other compound while maintaining its general structural features. Derivatives may be obtained for example by chemical functionalization and/or derivatization.
"Drug" as used herein refers to an active compound.
"Fluidizing conditions" refers to conditions which are given when a solid particle bed is passed through by a gas stream moving the entire mass of the solid material upwards until the lifting forces and gravity forces are in the statistical balance. This leads to a suspension of solid material in a gas phase where the solid particles can move freely in all dimensions. The result is a fluid-like system of a density between bulk density of the solid material in rest and the density of the streaming gas.

As used herein, "formation of droplets" refers to the conversion of a material into fine particles or pellets by measures like pressure atomization, two fluid atomization, centrifugal droplets formation and nozzles like e.g. piezo droplet forming devices, sound activated-, magnetic- and electrostatic droplet forming devices.

As used herein, a "hydrophilic excipient" is a pharmaceutically acceptable, pharmacologically substantially inert material with hydrophilic properties. Hydrophilicity means that the hydrophilic excipient should be substantially water soluble.
"Lipid" refers to its conventional sense as a generic term encompassing fats, lipids, alcohol-ethersoluble constituents of protoplasm, which are insoluble in water. Lipids are composed of fats, fatty oils, essential oils, waxes, steroid, sterols, phospholipids, glycolipids, sulpholipids, aminolipids, chromolipids, and fatty acids. The term encompasses both naturally occurring and synthetic lipids. Preferred lipids in connection with the present invention are: steroids and sterol, particularly cholesterol, phospholipids, including phosphatidyl and phosphatidylcholines and phosphatidylethanolamines, and sphingomyelins. Where there are fatty acids, they could be about 12-24 carbon chains in length, containing up to 6 double bonds. The fatty acids are linked to a backbone, which may be derived from glycerol. The fatty acids within one lipid can be different (asymmetric), or there may be only 1 fatty acid chain present, e. g., lysolecithins. Mixed formulations are also possible, particularly when the non-cationic lipids are derived from natural sources, such as lecithins (phosphatidylcholines) purified from egg yolk, bovine heart, brain, liver, or soybean.
"Liposomes" are artificial lipid bilayer vesicles of various sizes and structures. Unilamellar vesicles are liposomes defined by a single lipid bilayer enclosing an aqueous space. In contrast, oligo- or multilamellar vesicles comprise several membranes. Typically, the membranes are roughly 4 nm thick and are composed of amphiphilic lipids, such as phospholipids of natural or synthetic origin. Optionally, the membrane properties can be modified by the incorporation of other lipids such as sterols or cholic acid derivatives. Liposomes with particularly flexible membranes based on phospholipids with a low phase transition temperature (i.e. below body temperature) are sometimes referred to as transfersomes.

Depending on their diameter and number of bilayer membranes, liposomes may also be classified as multilamellar vesicles (MLV, two or more bilayers, typically above approx. 150 to 200 nm), small unilamellar vesicles (SUV, one single bilayer, typically below about 100 nm), multivesicular vesicles (MVV, several vesicular structures within a larger vesicle), and large unilamellar vesicles (LUV, one single bilayer, typically larger than about 100 nm).
"Paclitaxel" (which should be understood herein to include analogues, formulations, and derivatives such as, for example, deacetylpaclitaxel, deacetyl-7-epipaclitaxel, docetaxel, taxotere (a formulation of docetaxel), 10-desacetyl analogs of paclitaxel and 3'N-desbenzoyl-3'N-t-butoxycarbonyl analogs of paclitaxel) may be readily prepared utilizing techniques known to those skilled in the art (see also WO 94/07882, WO 94/07881, WO 94/07880, WO 94/07876, WO 93/23555, WO 93/10076, US Pat. Nos. 5,294,637, 5,28-3,253, 5,279,949, 5,274,137, 5,202,448, 5,200,534, 5,229,529, and EP 590,267), or obtained from a variety of commercial sources, including for example, Sigma Chemical Co., St. Louis, Mo. (T7402 from Taxus brevifolia, or T-1912 from Taxus yannanensis). Paclitaxel should be understood to refer to not only the common chemically available form of paclitaxel, but analogs (e. g., taxotere, as noted above) and paclitaxel conjugates (e.g., paclitaxel-PEG, paclitaxeldextran, or paclitaxel-xylose).
"Particle" as used herein refers to an entity of solid matter. The particle may be in the size range of about 1 µm to about 5 mm. The matter is typically composed of different chemical elements, compounds or constituents. For example, a particle may comprise liposomes and excipients. The solid matter may be porous or non-porous. At a given temperature a "frozen particle" typically comprises solid constituents which are fluid at room temperature. Typically, these constituents are water and water miscible solvents. A "dry particle" comprises such constituents only in an amount of lower than 10% (w/w).

The term "taxane" as used herein refers to the class of antineoplastic agents having a mechanism of microtubule action and having a structure that includes the unusual taxane ring structure and a stereospecific side chain that is required for cytostatic activity. Also inciuded within the term "taxane" are a variety of known derivatives, including both hydrophilic derivatives, and hydrophobic derivatives. Taxane derivatives include, but not limited to, galactose and mannose derivatives described in International Patent Application No. WO 99/18113, piperazino and other derivatives described in WO 99/14209, taxane derivatives described in W0 99/09021, WO 98/22451, and US Patent No. 5,869,680, 6-thio derivatives described in WO 98/28288, sulfenamide derivatives described in U. S. Patent No. 5,821,263, and taxol derivatives described in US Patent No. 5,415,869.

As used herein, "thermally sensitive", "temperature-sensitive" or "thermally labile" means that a material is incompatible with drying methods based on the evaporation of water by heat, such as spray drying. In other words, a thermally labile material looses at least some of its structure, activity, functionality or chemical purity when treated by such thermal drying methods, so that the product may be pharmaceutically unacceptable.

Dry particles obtainable by the inventive method may have different sizes, shapes and compositions. Usually, the majority of the dry particles and/or pellets have an average diameter in the size range of about 1 µm to about 5 mm. Preferably, small dry particles have an average diameter of about 5 µm to about 500 µm, and preferably from about 10 µm to about 400 µm. More preferably, small dry particles have an average diameter of about 20 µm to about 300 µm, from about 30 µm to about 250 µm, or from about 35 µm to about 200 µm. Preferably, large dry particles have an average diameter of about 50 µm to about 5000 µm or from about 100 µm to about 3000 µm. According to another preferred embodiment, the large dry particles have an average diameter of about 200 µm to about 2000 µm, from about 500 µm to about 1500 µm, or from about 750 µm to about 1200 µm.

Small dry particles and large dry particles have different properties, which are favourable depending on the further processing of the particles like filling and packing. Large dry particles are less prone to abrasion and flow easier than small dry particles.

Average diameters of the particles can be measured and described in various different ways. According to the present invention, an average diameter of the particles refers to the number average diameter of a sample as measured by laser diffraction or an equivalent method, unless stated otherwise. The average diameter of a colloidal system is expressed as a number average diameter of a sample as measured by photon correlation spectroscopy or an equivalent method, unless stated otherwise.

An aqueous medium comprises water, preferably in an amount of at least 50% (vol/vol), more preferably in an amount of at least 70% (vol/vol) and most preferably in an amount of at least 90% (vol/vol). Besides water, the aqueous medium used in the present invention may comprise one or more further liquid constituents which are at least partially miscible with water, such as an alcohol (e.g. C₁₋₄ alcohols such as methanol, ethanol, propanol, butanol and combinations thereof, etc.) or a ketone (e.g. C₃₋₄ ketones such as acetone, methylethyl-ketone and combinations thereof, etc.). In a preferred embodiment, the aqueous medium is water or a mixture of water and ethanol.

In a preferred embodiment, the aqueous medium comprises a hydrophilic excipient. Useful hydrophilic excipients may be found among several chemical classes of compounds. According to a further preferred embodiment, the hydrophilic excipient is a saccharide, e.g. a mono-, di-, oligo- or polysaccharide, a sugar alcohol, an amino acid, a peptide, a protein, a water-soluble polymer, or a combination thereof.

A saccharide, or carbohydrate, is defined as a compound predominantly composed of carbon, hydrogen, and oxygen. Useful saccharides include sugar and sugar alcohols, oligosaccharides, water soluble polysaccharides and derivatives thereof. Preferred saccharides according to the invention include glucose, fructose, lactose, sucrose, trehalose, maltose, cellobiose, galactose, maltotriose, maltopentose, raffinose, dextrin, dextran, inulin, mannitol, sorbitol, xylitol, chitosan, water soluble cellulose derivatives such as methylcellulose, hydroxypropylcellulose, hydroxyethylcellulose, and hypromellose, alginates, soluble starches or starch fractions, xanthan gum, guar gum, pectin, carrageen, galactomannan, gellan gum, tragacanth, including any derivatives of these. Particularly preferred saccharides are glucose and trehalose.

Other useful hydrophilic excipients may be selected from other chemical classes, such as from water soluble amino acids, peptides or proteins. For example, glycine or other natural amino acids may be used. Useful proteins include gelatine, albumin, whey protein, soy protein, or other food or vegetable proteins.

Still further examples of useful hydrophilic excipients are polymers such as water soluble polymers such as solid polyethylene glycols, polyvinylalcohol, polyacrylates, or polyvinylpyrrolidone.

According to the invention, mixtures of more than one hydrophilic excipient may be used. For example, there may be a need to adjust several parameters such as pH, solubility, and wettability independently. In this case, a first hydrophilic excipient may be selected as a basic carrier material for the colloidal system, whereas one or more further hydrophilic excipients may be incorporated to obtain a certain pH and/or wettability.

The content of the hydrophilic excipient, or mixture of hydrophilic excipients, in the aqueous phase may vary widely, depending on its aqueous solubility and other considerations, and may be even up to about 80 wt.-%. More preferably, it is from about 0.1 wt.-% to about 65 wt.-%. A content of about 3 wt.-% to about 60 wt.-%, and particularly from about 5 wt.-% to about 50 wt.-%.

Of course, the aqueous medium may comprise still further excipients or auxiliary substances, which may or may not be hydrophilic or water soluble. Depending on their nature and that of the extraction medium, i.e. on whether or not these substances are soluble in and extracted by the extraction medium, such substances may be comprised in the dry particles or removed together with the water. Substances, which are comprised, should be pharmaceutically acceptable.

Further excipients which may be useful in particles formulations, and in particular in particles formulations which are used for reconstitution and optionally, parenteral administration, are generally known to pharmaceutical formulation scientists. Preferred further excipients include stabilisers, surfactants, wetting agents, bulking agents, lyophilisation aids, antioxidants, chelating agents, preservatives, osmotic agents, acidic or alkaline excipients for adjusting the pH, etc.

Among the preferred excipients according to the invention are stabilisers and antioxidants. Antioxidants may prevent the oxidation of an incorporated active compound, but also that of components of the colloidal system, in particular if lipids are used which are sensitive to oxidisation. Useful compounds include, for example, lipid-soluble antioxidants such as alpha-, beta-, and gamma-tocopherol, ubiquinol, lycopene, alpha- and beta-carotene, nordihydroguaiaretic acid, butyl hydroxyanisole, butyl hydroxytoluene, ethylenediamine tetraacetic acid, diethylenetriamine pentaacetic acid, desferal, p-hydroxybenzoic, vanillic, syringic, 3,4-dihydroxybenzoic, p-coumaric, ferulic, sinapic and caffeic acids, ascorbyl palmitate, carnosol, esculetin, esculin, fraxetin, fraxin, quercetin, morin, etc. Particularly preferred are alpha-tocopherol and ethylenediamine tetraacetic acid, including the pharmaceutically acceptable derivatives thereof. On the other hand, if chemically pure, semisynthetic or synthetic saturated lipids are used for composing the colloidal systems, no antioxidant may be needed.

Colloidal systems of the present invention may be solid, semisolid or liquid. Preferably, the colloidal system is based on lipids, lipoids and/or amphiphilic compounds. In a preferred embodiment, colloidal systems are liposomes, lipid complexes, solid lipid nanoparticles, lipoplexes, niosomes, micelles or mixed micelles, preferably liposomes and in a more preferred embodiment cationic liposomes.

Colloidal systems, preferably liposomes used within the context of the present invention may comprise neutral, anionic or cationic lipids. Neutral or anionic lipids may be selected from sterols or lipids such as cholesterol, phospholipids, lysolipids, lysophospholipids, sphingolipids or pegylated lipids with a neutral or negative net charge. Useful neutral and anionic lipids thereby include: phosphatidylserine, phosphatidylglycerol, phosphatidylinositol (not limited to a specific sugar), fatty acids, sterols, containing a carboxylic acid group for example, cholesterol, 1,2-diacyl-sn-glycero-3-phosphoethanolamine, including, but not limited to, 1,2-dioleylphosphoethanolamine (DOPE), 1,2-dihexadecylphosphoethanolamine (DHPE), 1,2-diacyl-glycero-3-phosphocholines, 1,2-distearylphosphosphatidylcholine (DSPC), 1,2-dipalmitylphosphosphatidylcholine (DPPC), 1,2-dimyristylphosphosphatidylcholine (DMPC), phosphatidylcholine preferably egg PC, soy PC and sphingomyelin. The fatty acids linked to the glycerol backbone are not limited to a specific length or number of double bonds. Phospholipids may also have two different fatty acids. Preferably the neutral and/or anionic lipids are in the liquid crystalline state at room temperature and they are miscible (i.e. a uniform phase can be formed and no phase separation or domain formation occurs) with a cationic lipid, in the ratio as they are applied. In a preferred embodiment the neutral lipid is 1,2-dioleylphosphosphatidylcholine (DOPC).

Preferred cationic lipids include N-[1-(2,3-dioleoyloxy)propyl]-N,N,N-trimethyl ammonium salts, e.g. the methylsulfate. Preferred representatives of the family of -TAP lipids are DOTAP (dioleoyl-), DMTAP (dimyristoyl-), DPTAP (dipalmitoyl-), or DSTAP (distearoyl-). Other useful lipids for the present invention may include: DDAB, dimethyldioctadecyl ammonium bromide, 1,2-diacyloxy-3-trimethylammonium propanes, (including but not limited to: dioleoyl, dimyristoyl, dilauroyl, dipalmitoyl and distearoyl, also two different acyl chains can be linked to the glycerol backbone), N-[1-(2,3-dioloyloxy)propyl]-N,N-dimethyl amine (DODAP), 1,2-diacyloxy-3-dimethylammonium propanes, (including but not limited to: dioleoyl, dimyristoyl, dilauroyl, dipalmitoyl and distearoyl, also two different acyl chain can be linked to the glycerol backbone), N-[1-(2,3-dioleyloxy)propyl]-N,N,N-trimethylammonium chloride (DOTMA), 1,2-dialkyloxy-3-dimethylammonium propanes, (including but not limited to: dioleyl, dimyristyl, dilauryl, dipalmityl and distearyl, also two different alkyl chain can be linked to the glycerol backbone), dioctadecylamidoglycylspermine (DOGS), 3β-[N-(N',N'-dimethylamino-ethane)carbamoyl]cholesterol (DC-Chol), 2,3-dioleoyloxy-N-(2-(sperminecarboxamido)-ethyl)-N,N-dimethyl-1-propanaminium trifluoro-acetate (DOSPA), β-alanyl cholesterol, cetyl trimethyl ammonium bromide (CTAB), diC14-amidine, N-*tert*-butyl-N'-tetradecyl-3-tetradecylamino-propionamidine, 14Dea2, N-(alpha-trimethylammonioacetyl)didodecyl-D-glutamate chloride (TMAG), O,O'-ditetradecanoyl-N-(trimethylammonio-acetyl)diethanolamine chloride, 1,3-dioleoyloxy-2-(6-carboxy-spermyl)-propylamide (DOSPER), N,N,N',N'-tetramethyl-N,N'-bis(2-hydroxylethyl)-2,3-dioleoyloxy-1,4-butanediammonium iodide, 1-[2-(acyloxy)ethyl]2-alkyl(alkenyl)-3-(2-hydroxyethyl)-imidazolinium chloride derivatives as described by Solodin et al. (Solodin et al., 1995), such as 1-[2-(9(Z)-octadecenoyloxy)ethyl]-2-(8(Z)-heptadecenyl-3-(2-hydroxyethyl)imidazolinium chloride (DOTIM), 1-[2-(hexadecanoyloxy)ethyl]-2-pentadecyl-3-(2-hydroxyethyl)imidazolinium chloride (DPTIM), 2,3-dialkyloxypropyl quaternary ammonium compound derivatives, containing a hydroxyalkyl moiety on the quaternary amine, as described e.g. by Felgner et al. (Felgner et al., 1994) such as: 1,2-dioleoyl-3-dimethyl-hydroxyethyl ammonium bromide (DORI), 1,2-dioleyloxypropyl-3-dimethyl-hydroxyethyl ammonium bromide (DORIE), 1,2-dioleyloxypropyl-3-dimetyl-hydroxypropyl ammonium bromide (DORIE-HP), 1,2-dioleyl-oxypropyl-3-dimethyl-hydroxybutyl ammonium bromide (DORIE-HB), 1,2-dioleyloxypropyl-3-dimethyl-hydroxypentyl ammonium bromide (DORIE-Hpe), 1,2-dimyristyloxypropyl-3-dimethyl-hydroxylethyl ammonium bromide (DMRIE), 1,2-dipalmityloxypropyl-3-dimethyl-hydroxyethyl ammonium bromide (DPRIE), 1,2-disteryloxypropyl-3-dimethyl-hydroxyethyl ammonium bromide (DSRIE), cationic esters of acyl carnitines as reported by Santaniello et al. (US 5,498,633), cationic triesters of phospahtidylcholine, i.e. 1,2-diacyl-sn-glycerol-3-ethylphosphocholines, where the hydrocarbon chains can be saturated or unsaturated and branched or non-branched with a chain length from C₁₂ to C₂₄. Preferably, the cationic lipids have two acyl chains, which may be identical or different.

In a further preferred embodiment, the colloidal system is a non-liposomal colloidal carrier system for active ingredients, such as a lipid complex, lipoplex, cochleat, micelle, mixed micelle, or lipid nanoparticle.

In still a further preferred embodiment, micelles and mixed micelles according to the present invention are particularly small colloidal structures with various shapes and a length or diameter of typically about 5 nm to about 100 nm, formed via association of amphiphilic molecules such as detergents. In contrast to liposomes, these structures are based on monolayers which are less stable and tend to disassemble upon dilution. Micelles are e.g. disclosed in WO 02/085337 and EP 0 730 860.

It is a preferred embodiment that the colloidal system comprises an active compound. The active compound may be incorporated within or associated with the colloidal system. In the case of liposomes, for example, a hydrophilic active compound may be encapsulated within the aqueous inner space of the liposomes, whereas a lipophilic active compound is usually associated with the membrane-forming lipids. In the case of lipid nanoparticles, lipophilic compounds are also easily incorporated within the matrix of such nanoparticles.

Colloidal systems are particularly suitable carriers for active compounds which are not easy to be delivered effectively, such as poorly soluble compounds, sensitive (including thermally labile) active compounds, peptides, proteins, and nucleic acids including DNA, RNA, iRNA, siRNA, or oligonucleotides. Other active compounds are those which are delivered more effectively within a colloid since the distribution of colloids in the organism after intravenous injection is more favourable with regard to efficacy or side effects than the administration of a solution of the respective compound. Species of these compounds are antimicrobial, antifungal, antiviral, and cytostatic or cytotoxic agents which include doxorubicin, mitoxanthrone, and amphotericin B.

Colloidal systems, e.g. liposomes, used within the context of the invention are especially suitable for the encapsulation of hydrophobic drugs such as taxanes, camptothecins, doxorubicin, michellamine B, vincristine, and cisplatin. Preferred examples are paclitaxel, docetaxel and camptothecins, which have a low solubility in water and are heat sensitive.

In a further preferred embodiment, the colloidal system is a cationic liposomal preparation, especially a preparation comprising DOTAP, DOPC and paclitaxel in a ratio of about 50:47:3. This formulation is also designated MBT-0206 or EndoTAG-1. EndoTAG-1 has a lipid content of 10 mM in a 10% m/m trehalose dihydrate solution. The production of EndoTAG-1 is disclosed in WO 2004/002468.

Various methods are commonly used to prepare liposomes and to incorporate active ingredients. These include the film method, sonication, detergent dialysis, ethanol injection, ether infusion, reverse phase evaporation, extrusion, and high pressure homogenisation. These methods, the resulting products and their properties are described in more detail e.g. in Kerby et al., Liposomes, in: Encyclopedia of controlled drug delivery, vol. 1, 461-492 (John Wiley & Sons, 1999).

These methods often result in a high level of residuals such as detergents or organic solvents which have to be removed. Methods-that avoid the use of organic solvents involve e.g. extrusion, particularly high pressure extrusion of MLVs through polycarbonate filters of controlled pore size, sonication or high pressure homogenisation of MLVs to SUVs.

Colloidal systems, particularly liposomes can be administered orally, transdermally, intravenously, intrabronchially, intramuscularly, intraocularly, subcutaneously and intraperitoneally. As a drug delivery system, liposomes can significantly change the pharmacokinetic and pharmacodynamic fate of a compound by enhancing drug uptake, delaying the loss of rapidly cleared drugs and reducing drug toxicity. They have widely been investigated for the delivery of chemotherapeutic agents for treatment of cancer, antimicrobial agents for treatments of bacterial, viral and parasitic diseases, and also for use as immunological adjuvants for delivery of vaccines.

Liposomal preparations which have been dehydrated for the purpose of prolonged storage have to be re-hydrated in suitable liquid system, typically in an aqueous medium. It has surprisingly been found that aqueous liposomal dispersions and other lipid-based colloidal systems can be dried by the method of the invention in such a way that they can easily be reconstituted, usually retaining their approximate particle size and properties. Since the method uses mild conditions, in particular low temperatures under vacuum and percolation, the method is extremely useful for drying thermally labile liposomes.

Colloidal systems, e.g. liposomes may be labile in various respects. Their physical structure may be highly sensitive to heat, so that a thermal drying method would lead to the disassembly of the liposomal membranes, to the loss of functionality or to the loss of incorporated or associated active compound. Depending on their composition, they may also be chemically labile to heat. For example, some lipids hydrolyse or oxidise rapidly in an aqueous environment at elevated temperatures.

It is an object of the present invention to provide an aqueous medium comprising a biopharmaceutical agent in step a). The biopharmaceutical agent might be a molecule derived from amino acids or nucleic acids, or derived from a virus, a bacterium or a cell. The molecule derived from amino acids may be a oligo- or polypeptide, a protein, especially a recombinant protein. Examples are antibodies, especially monoclonal antibodies, antibody fragments, or cytokines like an interferon or interleukin.

In step b) of the inventive method, the aqueous medium of step a) is frozen to obtain frozen particles. Freezing is preferably performed by forming droplets of the aqueous medium and contacting it with a cryogenic fluid while maintaining freezing conditions to obtain frozen particles.

The cryogenic fluid can be a cold gas or a cryogenic liquid. Cryogenic liquids are chilled liquids like argon, helium, hydrogen, nitrogen, oxygen, methane, carbon dioxide, nitrous oxide, isopentane, hexane, or ethanol and other fluids like hydrocarbon fluids or mixtures thereof. In a preferred embodiment nitrogen is used.

Formation of droplets can be performed in various different ways. For example, liquid or liquid-like materials such as those provided in step a) can be atomised by spraying them through a nozzle or atomizer. Large particles can be prepared by avoiding high atomization pressure or pressure during the formation of droplets from a liquid or liquid-like material with appropriate droplets forming devices. In general, droplets may be formed by the application of mechanical forces. Since the technology for droplet formation is analogous in their physical features, the terms "spraying", "atomizing" and "dripping" are used interchangeably.

According to a preferred embodiment, the aqueous medium of step a) is atomised in step b) into different droplet sizes, whereas freezing is performed thereafter.

To achieve larger droplets for the preparation of larger particles, different techniques can be used. Larger droplets can either be formed by using larger nozzle diameters, less atomization pressure, other nozzle geometries and combinations of such devices and parameters. Other droplet forming devices besides spray nozzles can be used like centrifugal dispensers, piezo droplet forming devices, sound activated-, magnetic- and electrostatic droplet forming devices.

Useful atomising nozzles are known to the skilled worker in the field. They include, for example, rotating disk nozzles, impinging jet nozzles, capillary nozzles, single orifice nozzles, ultrasonic nozzles of vibrating or pulsating type, two-fluid nozzles such as coaxial two-fluid nozzles etc.

It is a preferred embodiment that the droplets which are obtained in step b) are prechilled in a vaporized cryogenic fluid. After atomization, droplets fall through a cold vapour phase in order to form frozen particles. The prechilled particles are subsequently collected and frozen in a tank containing cryogenic fluid. Vaporization of the cryogenic fluid may be achieved by nebulizing the cryogenic fluid in a temperature-controlled device in which prechilling is performed and/or rinsing the walls of the compartment with the cryogenic fluid. Optionally, i. e. alternatively to prechilling the aqueous medium is atomized in close vicinity or directly into the cryogenic fluid.

Step b) of the inventive method can be performed in a temperature-controlled device, e.g. the inventive apparatus.

It is a feature of the present invention that the frozen particles are dehydrated thereafter in step c) under reduced pressure, preferably without thawing said particles. Under reduced pressure, water is removed from frozen particles by sublimation.

In a preferred embodiment, step b) and step c) are carried out in the same device, in a temperature-controlled device. For this purpose the cryogenic fluid is removed from the device after freezing and the percolation medium is added under reduced pressure. Alternatively, frozen particles are removed from the freezing device and transferred into a separate temperature-controlled drying chamber.

It is a feature of the invention that during the drying process, a percolation medium, preferably a gas, is passed through or over the frozen particles under non-fluidizing conditions to increase evaporation by the increased transport of moisture from the drying material. In contrast thereto, under fluidizing conditions the frozen particles are solubilized in a percolation medium stream, which leads to a permanent movement of the particles within the percolation medium. Although the particles in a fluidized bed are statistically in balance, it is typical for such a system that particles are accelerated and lifted upwards from the fluidized bed with the gas stream. A mandatory technical measure to control fluidized beds is therefore a filter unit above the fluidized bed to avoid continuous loss of material.

In contrast to these conditions, i.e. when a medium is percolated through particles under non-fluidizing conditions, the stream is directed through a particle bed in a way that no relevant amount of particles is moved or suspended. The percolation medium can extract volatile compounds from the particle/powder bed but the powder bed is not significantly moved by the medium stream. Non fluidizing conditions still apply when the drying chamber itself or movable devices like blades or baffles lead to a controlled movement of the powder bed. Preferably, the percolation medium is passed through the drying chamber throughout step c).

The drying process of step c) according to the invention is performed under reduced pressure. The reduction of pressure enables decreased drying times for thermally labile compounds. Reduced pressure can be applied simultaneously to the percolation of medium through or over the drying material. Due to a low volume flow of the percolating medium compared to fluidizing conditions, a high vacuum can be obtained with conventional vacuum pumps. To achieve a similar vacuum under fluidizing conditions, vacuum pumps with high power and energy consumption are required. In contrast to drying methods described by Leuenberger et al. (US 4,608,764, US 6,584,782) no elaborate filtering system is necessary to retain the fluidized particles and particles generated by abrasion in the drying chamber of the respective device. Thus, obstructing filtering devices and the complexity of the filtering system which is applied are significantly reduced in the inventive method.

Particularly useful percolation media according to the invention are gases, i.e. substances or mixtures of substances, which are gaseous at the operating conditions, or they have a suitable vapour pressure in order to provide sufficient flux for drying. This does not necessarily require, that the substances are at room temperature and at atmospheric pressure in the gaseous state. In a preferred embodiment, the operating conditions indicate a pressure lower than atmospheric pressure. Therefore, also substances which are liquid or solid at atmospheric pressure may be applied. Also substances, which are liquid or solid at the operation conditions and which have a vapour pressure sufficient to provide suitable flux to promote drying can be applied. Examples of useful substances include air, nitrogen, carbon dioxide, alkanes, alcohols, ethane, propane, nitrous oxide, argon, oxygen, methane, butanes, pentanes, nitrous oxide, sulphur hexafluoride, chlorofluorocarbons, fluorocarbons, ethers comprising two alkyl radicals which may be the same or different and which contain no more than 3 carbon atoms, carbon monoxide, helium, hydrogen, xenon, including mixtures of any of these. Particularly preferred gases are carbon dioxide, ethane, argon, xenon, air, and nitrogen, and mixtures of any of these. The presently most preferred gas is nitrogen. The medium can be percolated through the particles/pellets from the bottom to the top or from the top to the bottom.

Carbon dioxide used as percolation medium can be generated from dry ice. The dry ice can be stored in a chamber beneath the drying chamber, which is connected to the drying chamber by a valve which is used to adjust the volume flow of carbon dioxide. Dry ice does not only generate gaseous carbon dioxide, but also cools the gas and the drying device. In general, a percolation medium can also be derived by sublimation of a solid material or by evaporation of a liquid material at suitable conditions.

In a preferred embodiment, the percolation medium contains very low amounts of moisture. The medium can be dried prior to use. The percolation medium can be dried in a drying device.

In another preferred embodiment, the percolation medium is cooled in a cooling device prior to application. In the cooling device, residual-moisture can also be withdrawn from the percolation medium.

In an even further preferred embodiment, the percolation medium volume is selected in the range of about 0.1 g/l/h to about 1000 g/l/h. Preferably, it is selected in the region of about 0.2 g/l/h to about 500 g/l/h. More preferably, the percolation medium volume flow is selected in the region of about 1 g/l/h to about 100 g/l/h and most preferably in the range of 10 g/l/h to about 20 g/l/h.

Vacuum conditions, including temperature conditions of the equipment, must be defined in correlation with physical parameters. In a preferred embodiment, the vacuum pressure should be selected in the range below the vapour pressure of the aqueous medium over the frozen particles at a suitable respective temperature. Depending on the transport properties of the percolation medium, the typical temperature should be selected in the range of about -90°C to about 50°C. Consequently the preferred pressure, e.g. in a temperature-cooled device, is between about 0.01 mbar to about 800 mbar.

More preferably, the vacuum is selected in the region of about 0.5 mbar to about 50 mbar and the temperature of the jacketed temperature-cooled device in the region -20°C to about 35°C. Most preferably the vacuum should be selected in the range of about 0.8 mbar to about 6 mbar.

According to another preferred embodiment, the temperature is raised during percolation. As used herein, a raise of temperature means that the temperature is increased with the constant drying status of the particles. Preferably, the maximum temperature relates to a temperature which does not lead to a thawing of the material. More preferably, the increase in temperature is selected in the range of about -20°C to about 15°C.

It is a preferred embodiment of the invention, that the actual temperature of the drying particles can be controlled by a combination of conventional chilling/heating while cooling which is induced by evaporating water from the pellets.

In order to perform step c) of the present invention a temperature cooled device, e.g. the inventive apparatus, can be used which can be maintained at different temperatures whereby the percolation medium can be supplied under vacuum. To achieve a proper temperature control, the walls of the vessel which is used for drying the particles as well as the percolation medium may be heated or cooled. The apparatus can be agitated itself. Alternatively, the particles can be stirred with a variation of possible techniques to achieve a blend with increasing evaporation. The agitation of the material also avoids a temperature gradient within the material.

It is a preferred embodiment, that step b) and c) of the present invention may be carried out in a single device. In another preferred embodiment, the aqueous medium of step a) is frozen to obtain frozen particles in a first device, and the frozen particles dried in a subsequent step in a different second device.

The invention also refers to an apparatus for the preparation of dry particles from an aqueous medium comprising a colloidal system and/or a biopharmaceutical agent. The apparatus comprises a first chamber for obtaining frozen particles from an aqueous medium comprising a colloidal system and/or a biopharmaceutical agent and a second chamber for dehydrating the frozen particles under reduced pressure and under non-fluidizing conditions.

The first chamber preferably comprises a means for introducing and atomizing the aqueous medium, means for introducing and atomizing a cryogenic fluid, and optionally means for controlling the pressure and/or temperature in the first chamber. The second chamber preferably comprises means for introducing frozen particles, a reservoir for cryogenic fluid, means for supplying a percolation medium under non-fluidizing conditions and means for controlling the pressure and/or temperature in the second chamber. The second chamber may be surrounded by a temperature controllable jacket which allows the cooling or heating of the chamber and optionally a change of temperature during the drying procedure. An outlet in the wall of the chamber may be connected to a vacuum pump. The walls of the chamber also comprise means, preferably a valve, to introduce frozen particles into the chamber and to withdraw the dried product from the chamber.

A further embodiment refers to an apparatus for preparing dry particles from an aqueous medium comprising a colloidal system and/or a biopharmaceutical agent comprising:
a first upper and a second lower chamber connected to each other, wherein
   (a) the upper chamber is preferably conical, tapers downwardly and comprises
      (i) an opening for introducing and preferably atomizing the aqueous medium, e.g. an atomizing nozzle, and
      (ii) a means for introducing and preferably nebulizing a cryogenic fluid, e.g. positioned in the top of the chamber,
      (iii) a container being connected to the opening (i) by a conduit comprising a liquid pump,
      (iv) a supply for the cryogenic fluid being connected to means (ii), and
      (v) optionally a temperature controlling means, e.g. a temperature controllable jacket, and
   (b) the lower chamber comprises
      (i) a temperature controlling means, e.g. a temperature controllable jacket,
      (ii) an opening, e.g. positioned in the top part of the lower chamber, being connected to a vacuum pump,
      (iii) an opening connected to a supply for a percolation medium e.g. at the bottom of the chamber, wherein said connection preferably comprises means, e.g. a valve to restrict the flow, and
      (iv) an opening, e.g. a valve to withdraw product from the chamber.

Another embodiment refers to an apparatus for freezing an aqueous medium comprising a colloidal system comprising:
a first upper chamber and a second lower chamber connected to each other, wherein
   a) the upper chamber is preferably conical, tapers downwardly and comprises
      (i) an opening for introducing and preferably atomizing the aqueous medium, e.g. an atomizing nozzle positioned in a way that the atomized medium can fall into the lower chamber, and
      (ii) a means for introducing and preferably nebulizing a cryogenic fluid, e.g. positioned in the top of the chamber,
      (iii) a container being connected to the opening (i) by a conduit comprising a liquid pump,
      (iv) a supply for cryogenic fluid being connected to means (ii), and
      (v) optionally a temperature controlling means, e.g. a temperature controllable jacket, and,
   (b) the lower chamber comprises,
      (i) an opening, e.g. a valve to release cryogenic fluid from the chamber,
      (ii) a second opening, e.g. a valve to withdraw product from the chamber,
      (ii) optionally a temperature controllable means, e.g. a temperature controllable jacket.

Freezing the aqueous medium comprising a colloidal system yields frozen particles which may be dried in an apparatus for drying frozen particles, which is another embodiment of the current invention.

The apparatus for drying particles comprises a chamber, which comprises,
(i) an opening, e.g. a valve to load frozen particles into the chamber and to withdraw dried product from the chamber,
(ii) an opening, e.g. in the top of the chamber, being connected to a vacuum pump,
(iii) an opening connected to a supply for a percolation medium, e.g. at the bottom of the chamber, wherein said connection preferably comprises means, e.g. a valve to restrict the flow, and
(iv) a temperature controllable means, e.g. a temperature controllable jacket, and
(v) optionally a device for the agitation of particles, e.g. a stirrer.

Still a further embodiment refers to an apparatus for freeze-drying particles comprising
(i) a rotatable chamber comprising a drive, e.g. an electronic drive to rotate the chamber,
(ii) an opening, e.g a valve for introducing and/or removing material,
(iii) a temperature controlling means, e.g. a temperature controllable jacket,
(iv) an opening connected to a supply for a percolation medium,
(v) a distribution device, e.g: a porous insert at the wall of the chamber, to distribute the supplied percolation medium, and
(vi) an opening being connected to a vacuum pump.

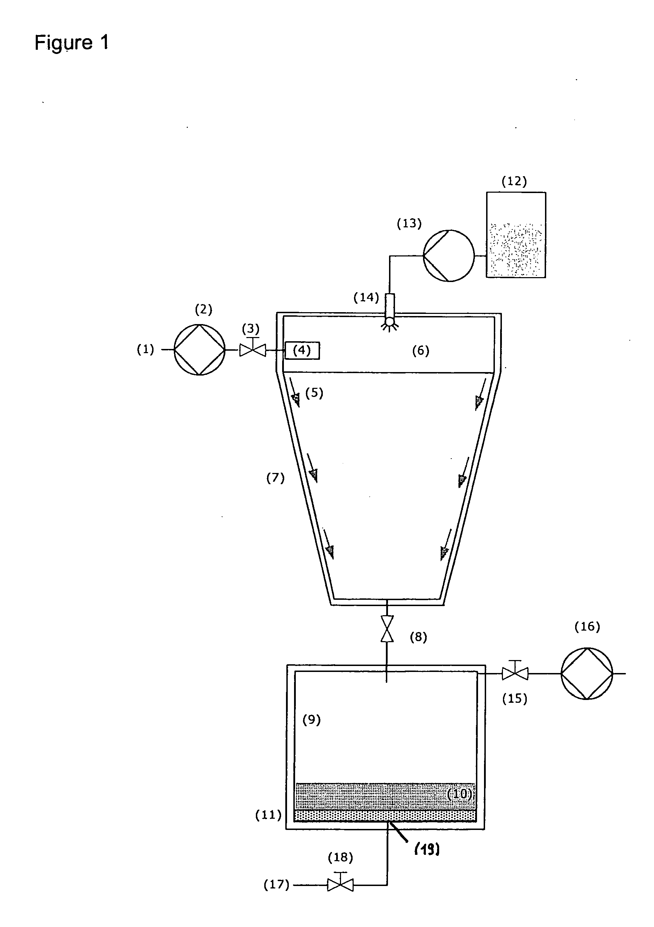

A preferred embodiment of an apparatus of the invention is shown in Figure 1. The apparatus comprises an upper chamber (7), e.g. a prefreezing vessel, and a lower chamber (9), e.g. a prechilled vacuum chamber, connected e.g. by a valve (8). Preferably, the upper chamber (7) has a conical shape tapering downwardly. Optionally, the walls of the upper chamber (7) are insulated and/or comprise a temperature controllable jacket.

The upper chamber (7) has means for nebulizing a cryogenic fluid (4), e.g. liquid nitrogen, and an atomization nozzle (14) for atomizing an aqueous medium. Optionally the nebulization means sprays cryogenic fluid against the wall of the chamber in a way that a free flowing film of cryogenic fluid is established. The means for nebulization is connected to a supply of cryogenic fluid (1). The atomization nozzle is connected to a container which comprises the aqueous medium (12) which is to be dried. To achieve the pressure which is needed for atomization, a suitable- pump (13) is supplying the aqueous medium to the nozzle (14). The nozzle is positioned in a way that the droplets resulting from the atomisation can fall through the valve (8) of sufficient diameter into the lower chamber (9) comprising cryogenic fluid.

In the lower chamber (9), the pre-frozen droplets are collected in a cryogenic fluid (10) and subsequently dehydrated. The lower chamber is surrounded by a temperature controllable jacket which allows the cooling or heating of the chamber and optionally a change of the temperature during the drying procedure. An outlet (15), e.g. a butterfly valve, is connected to a vacuum pump (16). For the drying process, the cryogenic fluid can be withdrawn from the chamber through the outlet (15) and the vacuum pump (16) or optionally via an additional valve. The cryogenic fluid is withdrawn from the device by increasing the temperature leading to the evaporation of the cryogenic fluid.

In order to pass a percolation medium through the frozen particles, the lower chamber (9) comprises an inlet (19), preferably at its bottom, which is connected to a percolation medium supply (17). The flow of the percolation medium can be controlled e.g. by a valve (18). To achieve an even percolation of the medium, a distribution device (11), e.g, a filter or sieve to distribute the flow over the bottom of the chamber may be positioned over said inlet (19). In a preferred embodiment, the distribution device (11) is a porous material or a perforated plate. Optionally, the lower chamber (9) comprises means, e.g. a second valve, to withdraw product from the chamber. The lower chamber can also comprise a device for the agitation of the pellets, preferably a stirrer.

The apparatus may also comprise means for measuring temperature and pressure in the different parts of the apparatus.

Further, the apparatus may comprise one or more conduits connecting a chamber to a pump, e.g. a nitrogen pump (2) or a vacuum pump (16). The conduits may comprise valves. (3, 15).

The inventive apparatus may also comprise a means for controlling the temperature of the percolation medium, or for drying the percolation medium, or both.

The vacuum pump of the apparatus may be connected to the supply of the percolation medium to constitute a circuit. Therein, the percolation medium which is withdrawn from the chamber is recycled for reuse.

Materials and technical components, which are suitable for constructing the described apparatus, are generally known to the expert in the field.

Freezing, percolating and vacuum drying of the aqueous medium under non-fluidizing conditions in order to obtain dry particles under the conditions specified above leads to the removal of water, i.e. to the drying of components contained in the aqueous medium, and to the formation of dry, solid particles of the hydrophilic excipient wherein the colloidal system or biopharmaceutical agent is comprised. Free flowable solid particles, which typically represent dry particles are obtained, which may be collected after reducing the vacuum pressure to approximately normal conditions.

Typically, the solid particles are substantially dry. If the process is conducted for a sufficiently long time or using a sufficient sublimation rate for the selected amount of aqueous medium to be dried, a residual water content of no more than about 5 wt.-%, and typically not more than about 4 wt.-% is easily achievable in only one drying step.

In order to obtain dry, solid particles in which a colloidal system is incorporated or embedded, it is useful to select a weight ratio of hydrophilic excipient to colloidal system which favours an acceptable degree of the colloid. For example, if the aqueous medium comprises a higher amount of colloidal system than hydrophilic excipient, a part of the colloidal system may not be incorporated into or embedded in the dry particles. Therefore, it is preferred that the weight ratio of hydrophilic excipient to colloidal system is at least about 1:1, and more preferably at least about 2:1, such as about 3:1 to about 5:1 or even higher. On the other hand, an extremely high ratio should be avoided since it might lead to a rather large powder (dry particles) volume for a certain content of colloidal system and thus to a large volume of liquid composition after reconstitution. Thus, ratios of less than about 50 and particularly of less than about 20 are considered useful and ratios of more than about 50 or even more than 100 are presently less preferred.

As pointed out, the method of the invention allows the conversion of an aqueous medium comprising a colloidal system or a biopharmaceutical agent into dry particles and/or pellets. The method is conducted at low temperatures, so that it is particularly advantageous for the drying and stabilisation of thermally labile material. In contrast to known methods, e. g. lyophilisation, the inventive method requires less time and is very cost-effective. Furthermore, it can be conducted as a batch process, but also as a semi-continuous or continuous process. In contrast to lyophilisation, which often leads to a porous, coherent solid product, the inventive method leads to free-flowing particles like a powder which may be easily filled into primary packaging containers by standard - optionally aseptic - powder filling processes.

Dry particles, or a powder comprising the same, obtainable by the method of the invention can be used in the manufacture of a medicament or a diagnostic product. The dry particles can be produced in a way to fulfil all requirements of a pharmaceutical dosage form, may be used as such or filled into appropriate primary packaging containers. Alternatively, the inventive particles may be further processed. For example, they may be mixed with further active and/or inactive ingredients such as pharmaceutically acceptable carriers, excipients or solvents or may be compressed into-a pharmaceutical tablet.

Preferably, the inventive particles are used - either alone or with further constituents - for the manufacture of a pharmaceutical composition which can be in powder form, particularly in form of a sterile powder for reconstitution with an appropriate aqueous medium.

After reconstitution of the inventive particles, e. g. with water for injection or a buffer system, the resulting formulations can be used for parenteral (e.g. intravenous or locoregional) injection, oral administration, pulmonary or nasal inhalation. In case of preparing dry particles and/or pellets from an aqueous medium comprising liposomes, the liposomes substantially have the same average particle diameter (preferably up to 30% alteration or less) and/or substantially the same polydispersity index (preferably up to 50% increase or less) after reconstitution compared to the respective values before reconstitution.

Organic solvents, in case they are being used during preparation, will be extracted during the inventive method below permissible or detectable limits, which is a particular advantage of the invention over methods known in the prior art.

All journal articles, other references, patents and patent applications which are identified herein are incorporated by reference in their entirety.

### EXAMPLES

The following examples are meant to illustrate the invention and some of its preferred embodiments further without limiting its scope. Further examples and embodiments will easily be derived from the description, optionally in combination with generally known technical information.

### Liposome preparation

Cationic liposomes with a total lipid content between 10 mM and 40 mM were prepared by ethanol injection and extrusion through a polycarbonate membrane. DOTAP (1,2-dioleoyl-3-trimethylammonium-propane) and DOPC (1,2-dioleoyl-sn-glycero-3-phosphocholine), both from Avanti Polar Lipids (Alabaster, USA), were dissolved in ethanol. Multilamellar liposomes formed spontaneously upon the injection into 10% trehalose solution (Ferro Pfanstiehl, USA). The polydisperse liposome preparation was extruded five times through polycarbonate membranes of 200 nm pore size in order to obtain unilamellar liposomes with a defined size distribution.

### Analytics of the liposomes

The particle size (Z-average) and polydispersity (PI) of the liposomes were analyzed by photon correlation spectroscopy (PCS) using a Malvern Zetasizer Nano (Malvern Instruments, UK). The lipid concentration of DOTAP and DOPC was analyzed by HPLC using an UVNIS detector at 205 nm. Separation and quantification of the components was carried out using a C8 LiChrospher 60 RP-selected B column (250 x 4mm, 5 µm particle size) with a C18 pre-column.

### Particle preparation

In the experimental set-up an aqueous medium comprising liposomes is added under described technologies into a cryogenic fluid. The aqueous medium was atomized over variations of nozzles. After freezing the aqueous droplets in a cryogenic fluid percolation vacuum drying is performed. The particles and/or pellets size can be controlled by the atomization forces.

### Analytics of particles

Residual moisture was determined by a coulometric Karl Fischer titrator with a head-space oven (Analytic Jena AG, Germany). Particle size distribution was measured with a He-Ne laser beam equipped laser diffraction analyzer (Mastersizer X, Malvern, Germany).

### Example 1

An aqueous dispersion of preformed liposomes was prepared with a lipid concentration of 10 mM and a trehalose content of 10 wt.-%. The number average diameter of the liposomes was about 155 nm, with a polydispersity of about 0.21. The aqueous dispersion was sprayed using a nozzle having an orifice of 0.5 mm diameter at a 5 ml/min flow rate. The liposomal suspension was frozen over a pre-freezing chamber of gaseous nitrogen with subsequent freezing in the cryogenic fluid.

After freezing the particles, a vacuum was applied to the pre-chilled apparatus. The temperature of the vacuum chamber was raised from -40 to 15°C over a time period of 4 hours. The percolation medium, gaseous nitrogen, was supplied with a volume flow of 14.8 litres per hour through the pellets and/or particles. A vacuum was maintained between 4 and 5 mbar during the percolation drying process. The aqueous solvent was evaporated during the vacuum percolation drying. Then the pressure was slowly increased to atmospheric conditions. The vacuum chamber was opened and particles of solid structure were obtained. The residual moisture content was below 3 wt.-%. The size of the smooth and hollow particles was in the range of 500 to 3000 µm. After reconstitution with water for injection a liposomal dispersion was obtained. The number average particle diameter was about 151 nm, with a polydispersity of 0.26.

### Example 2

The method was conducted in analogy to Example 1, except that the orifice nozzle had a diameter of 0.2 mm at a 3 ml/min flow rate. The temperature of the vacuum chamber was raised from -8 to 14°C over a time frame of four hours. The percolation medium, gaseous nitrogen, was supplied with a volume flow of 22.2 litres per hour. The vacuum was maintained between 3 and 5 mbar during the percolation vacuum drying process. Dry particles were obtained with particle sizes varying between 200 to 1600 µm. The residual moisture content was below 5 wt.-%. After reconstitution of the powder, liposomes were obtained with a number average diameter of 157 nm and a polydispersity of 0.24.

### Example 3

The method was conducted in analogy to Example 2, except that the temperature of the vacuum chamber was raised from -8 to 10°C over a time frame of twelve hours. The percolation medium, gaseous nitrogen, was supplied with a volume flow of 14.8 litres per hour. The vacuum was maintained between 3 and 4 mbar during the percolation vacuum drying process. Dry particles were obtained with particle sizes varying between 300 to 2000 µm. The residual moisture content was below 4 wt.-%. After reconstitution of the powder, liposomes were obtained with a number average diameter of 163 nm and a polydispersity of 0.24.

### Example 4

The method was conducted in analogy to Example 1, except that the aqueous dispersion was sprayed using a two-fluid nozzle having an orifice of 0.75 mm diameter at a 3 ml/min flow rate. The temperature of the vacuum chamber was raised from -1 to 9°C over a time frame of four hours. The percolation medium, gaseous nitrogen, was supplied with a volume flow of 14.8 litres per hour. The vacuum was maintained between 2 and 4 mbar during the percolation vacuum drying process. Dry particles were obtained with particle sizes varying between 100 to 800 µm. The residual moisture content was below 4 wt.-%. After reconstitution of the powder, liposomes were obtained with a number average diameter of 153 nm and a polydispersity of 0.23.

### Example 5

The method was conducted in analogy to Example 1, except that the aqueous dispersion was sprayed using a two-fluid nozzle having an orifice of 0.75 mm diameter at a 7 ml/min flow rate. The temperature of the vacuum chamber was raised from -1 to 9°C over a time frame of two hours. The percolation medium, gaseous nitrogen, was supplied with a volume flow of 29.6 litres per hour. The vacuum was maintained between 2 and 3 mbar during the percolation vacuum drying process. Dry particles were obtained with particle sizes varying between 50 to 900 µm. The residual moisture content was below 3 wt.-%. After reconstitution of the powder, liposomes were obtained with a number average diameter of 159 nm and a polydispersity of 0.24.

### Example 6

The method was conducted in analogy to Example 5. The percolation medium, gaseous nitrogen, was supplied with a volume flow of 22.2 litres per hour. The vacuum was maintained between 2 and 2.5 mbar during the percolation vacuum drying process. Dry particles were obtained with particle sizes varying between 60 to 800 µm. The residual moisture content was below 4 wt.-%. After reconstitution of the powder, liposomes were obtained with a number average diameter of 138 nm and a polydispersity of 0.25.

### Example 7

The method was conducted in analogy to Example 5, except that the percolation medium was supplied over the particles. The percolation medium, gaseous nitrogen, was supplied with a volume flow of 22.2 litres per hour. The vacuum was maintained between 2 and 2.5 mbar during the percolation vacuum drying process. Dry particles were obtained with particle sizes varying between 60 to 800 µm. The residual moisture content was below 4 wt.-%. After reconstitution of the powder, liposomes were obtained with a number average diameter of 140 nm and a polydispersity of 0.25.

### Example 8

The method was conducted in analogy to Example 7. The percolation medium, gaseous nitrogen, was supplied with a volume flow of 22.2 litres per hour. The vacuum was maintained between 1.5 and 3.0 mbar during the percolation vacuum drying process. Dry particles were obtained with particle sizes varying between 30 to 700 µm. The residual moisture content was below 3 wt.-%. After reconstitution of the powder, liposomes were obtained with a number average diameter of 117 nm and a polydispersity of 0.22.

### Example 9

The method was conducted in analogy to Example 7. The percolation medium, gaseous nitrogen, was supplied with a volume flow of 22.2 litres per hour. The vacuum was maintained between 2.5 and 3.0 mbar during the percolation vacuum drying process. Dry particles were obtained with particle sizes varying between 50 to 900 µm. The residual moisture content was below 4 wt.-%. After reconstitution of the powder, liposomes were obtained with a number average diameter of 143 nm and a polydispersity of 0.23.

### Example 10

The method was conducted in analogy to Example 7, expect that the temperature of the vacuum chamber was raised from -5 to 5°C over a time frame of three hours. The percolation medium, gaseous nitrogen, was supplied with a volume flow of 29.60 litres per hour. The vacuum was maintained between 2 and 3 mbar during the percolation vacuum drying process. Dry particles were obtained with particle sizes varying between 30 to 1000 µm. The residual moisture content was below 4 wt.-%. After reconstitution of the powder, liposomes were obtained with a number average diameter of 150 nm and a polydispersity of 0.22.

### Figure Legend

**Figure 1:** Apparatus for freezing a liquid and drying the frozen particles by percolation in a single device.
(1) Nitrogen supply
(2) Nitrogen pump
(3) Butterfly valve
(4) Nitrogen nebulization
(5) Liquid nitrogen
(6) Vaporized nitrogen
(7) Prefreezing vessel
(8) Valve
(9) Prechilled vacuum chamber
(10) Liquid Nitrogen
(11) Filter/sieve
(12) Suspension vessel
(13) Liquid pump
(14) Nozzle
(15) Butterfly valve
(16) Vacuum pump
(17) Percolation medium supply
(18) Ball valve
(19) Inlet for percolation medium

## Claims

1. A method for the preparation of dry particles from an aqueous medium comprising a colloidal system and/or a biopharmaceutical agent comprising the steps
a) providing an aqueous medium comprising a colloidal system and/or a biopharmaceutical agent,
b) freezing the aqueous medium of step a) to obtain frozen particles, and
c) dehydrating the frozen particles and/or pellets of step b) to obtain dry particles,
**characterized in that** step c) is performed by contacting the frozen particles under reduced pressure and under non-fluidizing conditions with a percolation medium.

2. The method of claim 1, wherein said percolation medium is passed through and/or over said frozen particles.

3. The method of claim 1 or 2, wherein step c) is performed without thawing the frozen particles.

4. The method of any one of claims 1-3, wherein said colloidal system comprises a colloidal drug carrier, preferably a liposome and most preferably a cationic liposome.

5. The method of any one of claims 1-4, wherein said colloidal system comprises an active compound.

6. The method of claim 5, wherein said active compound is an antimicrobial, antifungal, antiviral, and cytostatic or cytotoxic agent.

7. The method of claim 5 or 6, wherein said active compound is camptothecin or a taxane, preferably paclitaxel or docetaxel.

8. The method of any one of claims 1-7, wherein said colloidal system comprises DOTAP, DOPC and paclitaxel in a ratio of about 50:47:3.

9. The method of any one of claims 1-3, wherein said biopharmaceutical agent is a molecule selected from amino acids, peptides, proteins, nucleotides or nucleic acids.

10. The method of claim 9, wherein said molecule is a protein, preferably an antibody.

11. The method of any one of claims 1-10, wherein said dry particles have an average diameter between about 1 µm and about 5 mm.

12. The method of any one of claims 1-11, wherein said dry particles have an average diameter between about 100 µm and about 3 mm.

13. The method of any one of claims 1-12, wherein said aqueous medium comprises at least one further liquid constituent, preferably a volatile organic solvent.

14. The method of any one of claims 1-13, wherein said aqueous medium comprises at least one alcohol or ketone such as methanol, ethanol, propanol, butanol, acetone, methylethylketone, D11 F, D1150, or a mixture thereof.

15. The method of any one of claims 1-14, wherein said aqueous medium further comprises a hydrophilic excipient.

16. The method of claim 15, wherein said hydrophilic excipient is a saccharide, preferably trehalose.

17. The method of any one of claims 1-16, wherein said aqueous medium comprises a further excipient.

18. The method of any one of claims 1-17, wherein said aqueous medium is atomized previous-to step b).

19. The method of any one of claims 1-18, wherein step b) is performed by contacting the aqueous medium with a cryogenic fluid.

20. The method of claim 19, wherein said cryogenic fluid is selected from argon, helium, hydrogen, nitrogen, oxygen, methane, carbon dioxide, nitrous oxide, isopentane, hexane, ethanol or another fluid like an hydrocarbonic fluid, a fluorocarbon or a mixture thereof.

21. The method of claim 19 or 20, wherein said cryogenic fluid is liquid nitrogen.

22. The method of any one of claims 1-21, wherein step b) comprises the formation of droplets.

23. The method of claim 22, wherein said droplets are prechilled in a vaporized cryogenic fluid to obtain frozen particles.

24. The method of any one of claims 1-23, wherein step b) is performed in a temperature-controlled device.

25. The method of any one of claims 1-24, wherein step c) is performed in a temperature-controlled device.

26. The method of any one of claims 1-25, wherein step c) is performed continuously.

27. The method of any one of claims 1-25, wherein said percolation medium is carbon dioxide, ethane, argon, xenon, air, nitrogen or a mixture of any of these, preferably nitrogen.

28. The method of any one of claims 1-26, wherein said reduced pressure is below the vapour pressure of the aqueous medium over said frozen particles of step b) at the respective temperature.

29. The method of claim 28, wherein the temperature is raised during step c).

30. The method of claim 28 or 29, wherein the temperature is between about -90°C and 50°C.

31. The method of any one of claims 1-30, wherein the reduced pressure in step c) is between about 0.1 and 800 mbar, preferably between about 0,5 and 50 mbar.

32. An apparatus for preparing dry particles from an aqueous medium comprising a colloidal system and/or a biopharmaceutical agent comprising:
a first chamber for obtaining frozen particles from an aqueous medium comprising a colloidal system and/or a biopharmaceutical agent and a second chamber for dehydrating the frozen particles and/or pellets under reduced pressure and under non-fluidizing conditions.

33. An apparatus for preparing dry particles from an aqueous medium comprising a colloidal system and/or a biopharmaceutical agent comprising:
a first upper and a second lower chamber connected to each other, wherein
(a) the upper chamber comprises
(i) an opening for introducing and preferably atomizing the aqueous medium,
(ii) a means for introducing and preferably nebulizing a cryogenic fluid,
(iii) a container being connected to the opening (i) by a conduit comprising a liquid pump,
(iv) a supply for the cryogenic fluid being connected to means (ii),
(v) optionally a temperature controlling means, and
(b) the lower chamber comprises
(i) a temperature controlling means,
(ii) an opening connected to a vacuum pump,
(iii) an opening connected to a supply for a percolation medium, wherein said connection comprises means to restrict the flow, and
(iv) an opening to withdraw product from the chamber.

34. The apparatus of claim 32 or 33, comprising means to distribute the flow of the percolation medium in the bottom of the second chamber which is positioned over said outlet.

35. The apparatus of any one of claims 32-34, wherein said means to distribute the flow of percolation medium is a porous material or a perforated plate.

36. The apparatus according to any one of the claims 32-35, wherein said lower chamber comprises a valve to release percolation medium from the chamber.

37. An apparatus for freeze-drying particles and/or pellets comprising:
(i) a rotatable chamber comprising a drive to rotate the chamber,
(ii) an opening for introducing and/or removing material,
(iii)a temperature controlling means,
(iv)an opening connected to a supply for a percolation medium,
(v) a distribution device to distribute the supplied percolation medium, and
(vi)an opening being connected to a vacuum pump.

38. The apparatus according to any one of the claims 32-37, comprising devices for measuring temperature and pressure in the different parts of the apparatus.

39. The apparatus according to any one of claims 32-38, wherein one or more conduits connecting a chamber to a pump comprise a valve.

40. The apparatus according to any one of claims 32-39, comprising a device for controlling the temperature of the percolation medium and/or drying the percolation medium.

41. The apparatus according to any one of claims 32-40, wherein said vacuum pump is connected to the supply of a percolation medium to constitute a circuit.

42. A product obtainable by a method according to any one of claims 1-31.

43. A medicament obtainable by a method according to any one of claims 1-31.

44. The use of a product according to claim 42 for the manufacture of a medicament.
